# EUROPEAN PATENT APPLICATION

(11) **EP 2 255 793 A1**
(43) Date of publication of application: **01.12.2010**
(21) Application number: 09161448.7
(22) Date of filing: 28.05.2009
(51) Int. Cl.: A61K 9/20

(54) **Pharmaceutical composition comprising tamsulosin**

(71) Applicant: Krka Tovarna Zdravil, D.D., Novo Mesto, 8501 Novo Mesto (SI)
(72) Inventor: Sedmak, Gregor, 1000 Ljubljana (SI); Stefanic, Marko, 8340 Crnomejl (SI)
(74) Representative: UEXKÜLL & STOLBERG

(57) **Abstract**

The present invention relates to a pharmaceutical composition comprising tamsulosin or a pharmaceutically acceptable salt or solvate thereof in a solid dosage form and to a process for preparing such a composition.

## Description

The present invention relates to a solid pharmaceutical composition comprising tamsulosin or a pharmaceutically acceptable salt or solvate thereof and to a process for preparing such a composition.

Tamsulosin is the generic name of 5-[(2R)-2-[[2-(2-Ethoxyphenoxy)ethyl]amino]propyl]-2-methoxybenzenesulfonamide and is known as an α₁-adrenergic receptor antagonist. For many years, tamsulosin or a salt thereof and in particular its hydrochloride salt has been used in the treatment of benign prostatic hyperplasia (BPH). BPH itself may not cause any symptoms, but frequently leads to benign prostatic enlargement which can cause bladder outlet obstruction and may be associated with bothersome lower urinary tract symptoms (LUTS). Pharmaceutical formulations of tamsulosin are available in the form of a modified-release (MR) capsule formulation or an orally-controlled absorption system (OCAS^{®}) tablet formulation.

The tamsulosin MR capsule utilises the well-established multi-unit layer coated pellet technology. The pellets have a drug core and the MR characteristics are provided by the layer surrounding the pellets. In the gastrointestinal (GI) tract, the pellets are hydrated and the drug is released.

The above technology is dependent on the presence of water in the GI tract to release the active ingredient. Consequently, drug release is impeded during the passage of the formulation through the colon where the amount of water is limited. The OCAS^{®} tablet formulation was designed to overcome the low absorption from the colon resulting in a more constant 24-hour plasma concentration of tamsulosin without food effect.

For example, EP-A-0 661 045 is directed to adsorption system tablets and particularly describes sustained-release hydrogel preparations comprising (1) at least one drug, (2) a hydrophilic base ensuring penetration of water into the preparation and (3) a hydrogel-forming polymer. The hydrophilic base (2) is such that the amount of water required to dissolve 1 g of said hydrophilic base is not more than 5 ml at a temperature of 20 ± 5°C. Due to the use of said hydrophilic base the preparation undergoes a substantially complete gelation in the upper part of the GI tract, namely stomach and small intestine. The formed gel matrix is then maintained in the hydrated state in the colon and thus a sufficient drug release in the colon is achieved although water is hardly available there. This results in a uniform, sustained-release of the drug throughout the entire GI tract. To ensure that the release of the drug in the colon does not become insufficient, the amount of said hydrophilic base (2) may not be too small and EP-A-0 661 045 teaches that the amount of said hydrophilic base is generally about 5 to 80 wt.-%, based on the total weight of the preparation.

A preferred hydrogel-forming polymer according to EP-A-0 661 045 is polyethylene oxide and a preferred hydrophilic base according to EP-A-0 661 045 is polyethylene glycol. Accordingly, the currently marketed tamsulsoin OCAS^{®} tablet formulation comprises a high molecular weight polyethylene oxide as the hydrogel-forming polymer (Macrogol 7,000,000) and polyethylene glycol as hydrophilic base (Macrogol 8,000).

EP-A-1 523 994 describes that the manufacture of a tablet based on polyethylene oxide having a molecular weight of 2,000,000 or more as taught in EP-A-0 661 045 may be difficult. The polymer is said to become very sticky when exposed to moisture resulting in that it is difficult to handle during pulverization, granulation, tableting and the like, particularly during granulation. To address these manufacturing issues, EP-A-1 523 994 teaches a pharmaceutical composition for controlled-release which comprises a sized product comprising (a) a drug such as tamsulosin hydrochloride, (b) polyethylene oxdie with a high viscosity-average molecular weight of 2,000,000 or more, and (c) a size controlling agent. The size controlling agent is typically a portion of the polyethylene glycol used as the hydrophilic base. In particular, EP-A-1 523 994 describes to spray an aqueous solution or suspension of the size controlling agent onto the polyethylene oxide particles and to incorporate the obtained particles into a tablet by conventional techniques.

WO 2007/131804 proposes to avoid the use of polyethylene oxide as it suffers from bad tableting properties. It describes an alternative controlled-release pharmaceutical tablet comprising (a) 0.1 to 1 wt.-% of tamsulosin and (b) 40 to 80 wt.-% of a water-swellable matrix-forming composition comprising (i) a pH-sensitive swellable hydrophilic polymer, which is a cross-linked polyacrylic acid polymer such as carbomer, and (ii) a pH-insensitive swellable hydrophilic polymer such as hydroxypropyl methylcellulose.

EP-A-1 568 361 and EP-A-1 529 526 relate to sustained-release compositions of tamsulosin having a specific drug release profile or a specific ratio (Cₘᵢₙ/Cₘₐₓ) of the plasma tamsulsoin concentration, respectively. The sustained-release compositions can be in the form of a hydrogel-forming preparation, a preparation of an osmotic pump type, a gel preparation having combined a plurality of gums, a multi-layered tablet comprising a geometrically aligned drug layer and release-controlling layer(s), a gastroretentive dosage form using a swelling polymer or a matrix preparation using water-soluble polymers.

However, there is still a need for a stable pharmaceutical composition of tamsulosin or a salt or solvate thereof having a high drug content uniformity and a satisfactory drug release profile, which composition should not require the use of expensive excipients and should be obtainable by a simple and quick process.

These objects are surprisingly solved by the solid pharmaceutical composition according to claim 1 to 11 and 16. The invention also relates to the process according to claim 12 to 15.

The invention relates to a solid pharmaceutical composition which comprises
(a) granules comprising (i) at least one drug selected from tamsulosin or a pharmaceutically acceptable salt or solvate thereof, and (ii) hydrogel-forming polymer, and
(b) an extragranular phase comprising (iii) hydrogel-forming polymer.

The term "hydrogel-forming polymer" denotes any polymer which upon contact with an aqueous medium is capable of forming a hydrogel, i.e. a swollen, water-containing network of polymer chains that are water-insoluble. In particular, the hydrogel-forming polymer comprises hydrophilic groups that are capable of forming strong hydrogen bonds with water, such as -C-O-C-, -C-O-H, -C-N-C- or -C=O. Suitable examples of hydrogel-forming polymers include cellulose ethers like methylcellulose and ethylcellulose, sodium carboxymethyl amylopectin, sodium alginate, polyvinyl alcohol, polyvinyl pyrrolidone and polyethylene oxide.

In particular, the hydrogel-forming polymer (ii) and/or the hydrogel forming polymer (iii) are polyethylene oxide. Thus, the invention particularly relates to a solid pharmaceutical composition which comprises
(a) granules comprising (i) at least one drug selected from tamsulosin or a pharmaceutically acceptable salt or solvate thereof, and (ii) polyethylene oxide, and
(b) an extragranular phase comprising (iii) polyethylene oxide.

The component (i) of the composition is a drug selected from tamsulosin or a pharmaceutically acceptable salt or solvate thereof. Examples of useful salts are the addition salts with an inorganic or organic acid. Examples of useful inorganic acids include hydrochloric, hydrobromic, phosphoric and sulfuric acid. Suitable organic acids can be selected from methanesulfonic, acetic, citric, tartaric, maleic, and glucoheptanoic acid. Examples of suitable solvents for preparing solvates of tamsulosin include water, ethanol and isopropanol. Preferably, the drug (i) is tamsulosin hydrochloride. The drug (i) can be in a non-crystalline, i.e. amorphous, form or a crystalline form including any polymorphic or pseudopoly-morphic forms such as solvates like hydrates. Preferably, a crystalline form of the drug and most preferably crystalline tamsulosin hydrochloride is used.

Processes for preparing tamsulosin or a pharmaceutically acceptable salt or solvate thereof are known and for example described in EP-A-0 034 432, AT-A-397 960, EP-A-0 380 144, WO-A-03/035608 and WO-A-2005/063702.

The average particle size of tamsulosin used in the present invention can range from about 50 to 300 µm, more preferably 80 to 250 µm and most preferably 100 to 200 µm.

It is preferred that the composition comprises 0.02 to 5 wt.-%, more preferably 0.04 to 1 wt.-% and most preferably 0.08 to 0.8 wt.-% of drug (i).

It is also preferred that a single dosage form of the pharmaceutical composition according to the invention, such as a tablet, comprises 0.05 to 5 mg, preferably 0.1 to 1 mg and most preferably 0.2 and 0.8 mg of drug (i).

Typically, the components (ii) and (iii) of the composition according to the invention are polyethylene oxide. Preferably, the polyethylene oxide (ii) and/or the polyethylene oxide (iii) have an average molecular weight of at least 100,000, preferably of at least 600,000, more preferably of at least 2,000,000 and most preferably of about 7,000,000 or higher such as 10,000,000, based on dilute viscosity measurement. Typically, commercial grades of polyethylene oxide having a molecular weight in the range of about 100,000 to about 10,000,000 are useful as polyethylene oxide (ii) and (iii) of the composition according to the invention. According to one embodiment, polyethylene oxide (ii) has substantially the same molecular weight as polyethylene oxide (iii). In another embodiment, the molecular weight of polyethylene oxide (ii) is different from that of polyethylene oxide (iii).

It is preferred that the composition according to the invention comprises 50 to about 99 wt.-%, more preferably 80 to about 99 wt.-% and most preferably about 90 to about 98 wt.-% of polyethylene oxide (ii) and (iii), based on the total weight of the composition. According to one embodiment, the composition contains 96 to 98 wt.-% of polyethylene oxide (ii) and (iii), based on the total weight of the composition.

In a particularly preferred embodiment, the amount of polyethylene oxide (ii) contained in granules (a) is lower than the amount of polyethylene oxide (iii) of extragranular phase (b), that is the major portion of polyethylene oxide contained in the pharmaceutical composition according to the invention is not granulated. The weight ratio of polyethylene oxide (ii) to polyethylene oxide (iii) is typically about 1:200 to about 1:3, preferably about 1:100 to about 1:10, and more preferably about 1:50 to about 1:20.

As mentioned above, polyethylene oxide is a known hydrogel-forming polymer for preparing matrix-type sustained release formulations. However, its tendency to form a very strong gel upon contact with water may result in certain problems.

Firstly, if the pharmaceutical formulation is prepared by aqueous wet granulation of polyethylene oxide, very hard and compact granules are obtained resulting in technical problems during sieving of the dried granulate. As a consequence, such granules need to be milled so as to reduce their particle size and to make them useful for tableting.

Moreover, when a tablet prepared by granulation of polyethylene oxide comes in contact with water in the upper GI tract of a patient an extremely compact gel is formed, which is basically impenetrable for additional amounts of water. As a result, only the surface of the tablet is in the form of gel, while the inner core of the tablet remains dry. Thus, the drug release from such a tablet is deteriorated. In order to prepare a tablet with constant and uniform release of the drug over prolonged periods of time, the tablet has to undergo a gelling as complete as possible and as fast as possible.

As described above, EP-A-0 661 045 proposes to solve the problem associated with the poor gelling properties of granulated polyethylene oxide by including a considerable amount of a hydrophilic base, i.e. a highly water-soluble excipient such as polyethylene glycol. Due to its high solubility and relatively low molecular mass, in the upper GI tract the hydrophilic base dissolves rapidly on the surface of the tablet and diffuses away from it. The dissolved hydrophilic base leaves a tiny channel allowing additional amount of water to enter the tablet.

It has now surprisingly been found that it is possible to prepare a sustained-release dosage form which has very good gelling properties and a good drug content uniformity without the use of a water-soluble hydrophilic base, namely by providing a composition comprising granules (a) as inner phase and an extragranular phase (b). The granules (a) comprise the drug (i) and hydrogel-forming polymer (ii), such as polyethylene oxide, wherein the amount of (ii) in granules (a) is only a part of the complete amount of hydrogel-forming polymer used in the final pharmaceutical composition.

Due to the use of granules (a) comprising the drug granulated with hydrogel-forming polymer, such as polyethylene oxide, it is possible to achieve a sufficient content uniformity of the drug which appears to be important in view of the typically used low-dosage formulations of tamsulosin. On the other hand, as only a portion of the complete amount of hydrogel-forming polymer, such as polyethylene oxide, is comprised in granules (a) and the major portion of hydrogel-forming polymer, such as polyethylene oxide, is not granulated, the pharmaceutical composition according to the invention does not contain large, hard and compact granules. As a consequence, water in the upper GI tract of a patient has sufficient space to penetrate into the tablet resulting in improved gelling properties and, therefore, improved drug release properties.

Consequently, the pharmaceutical composition according to the invention is a solid sustained-release hydrogel-forming composition of tamsulosin having a good drug content uniformity and a satisfactory drug release profile. Furthermore, the composition can be prepared by a simple process using a hydrogel-forming polymer such as polyethylene oxide as main excipient. The use of a milling step as well as the use of additional excipients such as a hydrophilic base, a size controlling agent or a combination of a cross-linked polyacrylic acid polymer and a pH-insensitive swellable hydrophilic polymer can be avoided.

In one embodiment, the average particle size of granules (a) ranges from about 30 µm to about 1,000 µm, preferably from about 150 µm to about 600 µm. In particular, the average particle size can be determined by laser diffraction analysis.

In order to achieve the desired sustained-release of drug (i), it is preferred that extragranular phase (b) shows swelling properties such that the amount of water penetrating into extragranular phase (b) in the upper GI tract allows sufficient absorption of drug (i) in the colon. In particular, the swelling capacity of the composition according to the invention is such that the swelling ratio [(D₀ - D_{obs}) / D₀], with Do being the initial diameter of a sample tablet and D_{obs} being the diameter of the portion of the sample tablet not being swelled after 2 hours, is more than 0.4, preferably more than 0.5, more preferably more than 0.6 such as more than 0.7, when measuring a round 8 mm tablet in 250 ml of 0.5M phosphate buffer solution pH 6.8 at a paddle speed of 50 rpm.

Furthermore, it is preferred that extragranular phase (b) is obtainable by compressing hydrogel-forming polymer (iii), such as polyethylene oxide, and optionally further excipients at a compression pressure ranging from about 1 to about 30 kN such as less than 15 kN. Preferably, the hardness of extragranular phase (b) is about 50 to about 150 N, such as about 100 N.

In a particular embodiment, the composition according to the present invention preferably comprises none at all or only a very small amount, i.e. less than 10 wt.-%, more preferably less than 5 wt.-%, such as 0 to 4 wt.-%, most preferably less than 3 wt.-%, such as 0 to 2 wt.-%, of an additive selected from the group consisting of polyethylene glycol having an average molecular weight of less than about 20,000 g/mol, hydroxypropyl methylcellulose of 2 to 15mPa·s (2% w/v in H₂O), methylcellulose of 2 to 15mPa·s (2% w/v in H₂O), cross-linked polyacrylic acid polymer such as carbomer, and hydrophilic base ensuring penetration of water into the preparation which hydrophilic base has such a solubility that the amount of water for dissolving 1 g of the additive is not more than 5 ml at a temperature of 20°C. Examples of such hydrophilic bases are described in EP-A-0 661 045 and include highly hydrophilic polymers such as polyethylene glycol and polyvinylpyrrolidone, sugar alcohols such as D-sorbitol and xylitol, sugars such as sucrose, anhydrous maltose, D-fructose, dextran, glucose etc., surfactants such as polyoxyethylene-hydrogenated castor oil, polyoxyethylene-polyoxyproylene glycol, polyoxyethylene-sorbitan high molecular fatty acid ester etc., salts such as sodium chloride, magnesium chloride etc., amino acids such as glycine, β-alanine, lysine hydrochloride etc., and amino sugars such as meglumine.

The granules (a) and/or the extragranular phase (b) of the composition according to the invention can comprise additional excipients. Examples of such additional excipients include, but are not restricted to excipients selected from stabilizing agents, lubricants and glidants.

Preferably, granules (a) comprise at least one stabilizing agent. In particular, the stabilizing agent is selected from iron oxides, butylated hydroxytoluene, ascorbic acid, gallic acid, caffeic acid, flavonoids, isoflavonoids such as rutin and quercetin, or mixtures thereof.

In addition, it is preferred that the extragranular phase (b) comprises at least one lubricant. Suitable lubricants are preferably selected from stearic acid, stearic acid salts such as magnesium stearate, palmitic acid salts such as magnesium palmitate, oleic acid salts such as magnesium oleate, sodium lauryl sulfate, magnesium lauryl sulfate, hydrogenated vegetable oil, hydrogenated castor oil, talc, sodium stearyl fumarate, macrogols or mixtures thereof. Preferably, the lubricant is selected from stearic acid, magnesium stearate and hydrogenated vegetable oil.

Moreover, the extragranular phase (b) preferably comprises at least one glidant. Examples of suitable glidants include tribasic calcium phosphate, powdered cellulose, colloidal silicon dioxide, magnesium oxide, magnesium silicate, magnesium trisilicate, starch, talc and mixtures thereof. Preferably, the glidant is selected from colloidal silicon dioxide, magnesium trisilicate and talc.

Optionally, the composition according to the invention comprises at least one coating. Preferably, the coating comprises a polymer selected from the group consisting of water insoluble or soluble cellulose derivatives such as ethylcellulose, polyvinyl acetate, methacrylic ester copolymers, ammonio methacrylate copolymers, aminoalkyl methacrylate copolymers, methacrylic acid copolymers, polyvinyl alcohol, methylcellulose, hydroxypropyl methylcellulose and mixtures thereof.

Further, the coating can comprise at least one other excipient selected from the group consisting of plasticizers, antitacking agents, release rate modifiers, colorants, opacifiers, pigments, vehicles and mixtures thereof.

Preferably, the composition according to the invention is in the form of a tablet. The tablet can be coated with an appropriate material used for film coating, e.g. as described in Pharmaceutical Coating Technology (G. Cole (ed.), 1995). Film coating formulations usually contain components like polymer(s), plasticizer(s), colorant(s)/opacifier(s), vehicle (s) . In film coating suspension minor quantities of flavors, surfactants and waxes can be used. Film coating suspensions can also be used as ready-to-make preparations which are available on the market. In an alternate embodiment, the composition according to the invention is in the form of capsules or sachets.

According to a preferred embodiment, the composition according to the invention comprises
(a) granules comprising (i) tamsulosin hydrochloride, (ii) polyethylene oxide, and a stabilizing agent, and
(b) an extragranular phase comprising (iii) polyethylene oxide, a lubricant and a glidant.

According to a particularly preferred embodiment, the composition according to the invention comprises
(a) granules comprising (i) tamsulosin hydrochloride, (ii) polyethylene oxide, and butylated hydroxytoluene, and
(b) an extragranular phase comprising (iii) polyethylene oxide, magnesium stearate and colloidal silicon dioxide.

It is particularly preferred that the composition according to invention comprises, based on the total weight of the composition,
(a) granules comprising (i) 0.08 to 0.8 wt.-% of tamsulosin hydrochloride, (ii) 1 to 10 wt.-%, preferably 2 to 5 wt.-%, of polyethylene oxide, and 0.01 to 0.05 wt.-% of butylated hydroxytoluene, and
(b) an extragranular phase comprising (iii) 90 to 98 wt.-% of polyethylene oxide, 0.1 to 3 wt.-% of magnesium stearate and 0.1 to 3 wt.-% of colloidal silicon dioxide.

The invention also relates to a process for preparing the composition according to the invention comprising
(a) granulating (i) at least one drug selected from tamsulosin or a pharmaceutically acceptable salt or solvate thereof, (ii) hydrogel-forming polymer, preferably polyethylene oxide, and optionally further excipients to obtain granules,
(b) mixing the granules obtained in step (a) with (iii) hydrogel-forming polymer, preferably polyethylene oxdie, and optionally further excipients to obtain a mixture,
(c) compressing the mixture obtained in step (b) to form a solid pharmaceutical composition, and
(d) optionally coating the solid pharmaceutical composition obtained in step (c) with a coating material.

In step (a), components (i) and (ii) and optionally other excipients are converted into granules. This can be done by any known process such as wet or dry granulation. It is preferred that step (a) is performed by wet granulation. More preferably, wet granulation is performed using water or organic solvents such as alcohols like methanol or ethanol, or mixtures thereof as granulation liquid. Preferably, the granulation liquid is ethanol or a mixture of ethanol and water. In one embodiment, components (i) and (ii) are granulated using a solution of a stabilizing agent in ethanol. For example, wet granulation can be carried out in a high shear mixer. Prior to granulation step (a), the components to be converted into granules, e.g. drug (i), polyethylene oxide (ii) and optionally other excipients, can be thoroughly mixed to ensure a high homogeneity of the obtained granules.

At the end of step (a), the obtained granules are optionally dried and sieved, e.g. by using a 1 mm sieve, thus obtaining granules having a preferred average particle size of less than 1 mm such as 30 to 1,000 µm.

In step (b), the granules obtained in step (a) are mixed with hydrogel-forming polymer (iii), preferably polyethylene oxide, and optionally other excipients such as lubricants and glidants. In one embodiment, the granules and polyethylene oxide (iii) are firstly mixed with a glidant to obtain a pre-mix and said pre-mix is then mixed with a lubricant to obtain a mixture ready for compression. In another embodiment, mixing of step (b) is performed in one single step. In yet another embodiment, mixing (b) is performed in at least two steps with gradual adding of polyethylene oxide (iii). Mixing (b) can be carried out using a biconic mixer, a high shear mixer or any other suitable mixer.

In step (c), the mixture obtained in step (b) is compressed to obtain a solid pharmaceutical composition which is preferably in the form of a tablet. Tablet compression equipment known from the state of the art can be used for this step, such as a high speed rotary machine. In a preferred embodiment, the mixture of step (b) is compressed using a compression pressure of about 1 to about 30 kN, preferably less than 15 kN. Preferably, the obtained tablet has a diameter from 1.5 to 12 mm, preferably 2 to 10 mm, more preferably 4 to 8 mm and a weight 40 to 800 mg, preferably 50 to 600 mg and more preferably 80 to 400 mg. The shape of tablets can be of any known form, oblong and round shape being preferred in order to alleviate the swallowing of the tablet by patients having problems with swallowing, especially geriatric patients.

In step (c), the mixture obtained in step (b) can also be compressed to form spherical particles which can be filled into capsules or sachets.

In step (d) the composition obtained in step (c) can be coated with a coating material. Tablet coating equipment known from the state of the art can be used for this step. Water, organic solvents such as ethanol, acetone, isopropanol or mixtures thereof can be used for preparing coating dispersions.

The invention is also directed to a pharmaceutical composition obtainable by the above process according to the invention.

The pharmaceutical compositions of the present invention can be packaged in accordance with procedures and materials known from the state of the art. For example, as packaging material blisters, glass bottles, containers made of polymeric materials with suitable closure systems may be used. Packing can be performed under normal atmosphere or optionally under an atmosphere having a reduced oxygen concentration, preferably in an inert atmosphere or under reduced relative humidity such as 40% RH, preferably 35% RH and most preferably below 30% RH. Reduced oxygen concentration means that the concentration of oxygen in the gas surrounding the solid composition in the primary packaging is below 18 vol/vol%, preferably below 10 vol/vol% and most preferably below 5vol/vol%.

Decreased moisture permeability of primary packaging material is preferred in order to diminish moisture sorption by tamsulosin containing dosage forms to avoid any changes in drug stability. Low moisture permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, dosage forms containing tamsulosin or its pharmaceutically acceptable salts can be packed into primary packaging with desiccant. Desiccant can be placed inside the packaging unit together with tamsulosin dosage units such as tablets and/or in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers. An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. Examples of commercially available absorbers are Ageless™ (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

If the drug of the present composition is exhibited to a reduced oxygen partial pressure, the composition is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister.

Final packaging can optionally contain dessicant which can be used in blisters made of aluminium foil, incorporated into closure system of glass and/or polymeric containers or added directly into containers.

The invention is further illustrated by reference to the following examples which are not intended to limit the scope of the invention.

### Examples

| | **Example 1** | **Example 2** |
|---|---|---|
| | (mg/tablet) | (mg/tablet) |
| **Granules:** | | |
| Tamsulosin hydrochloride | 0.4 | 0.4 |
| Polyethylene oxide | 10.0 | 4.0 |
| Butylated hydroxytoluene | 0.04 | 0.04 |
| | | |
| **Extragranular phase:** | | |
| Polyethylene oxide | 186.1 | 192.1 |
| Colloidal silicon dioxide | 1.5 | 1.5 |
| Magnesium stearate | 2.0 | 2.0 |

Tamsulosin hydrochloride and the granular part of polyethylene oxide (average molecular weight: 7,000,000) were granulated in a high shear mixer with a solution of butylated hydroxytoluene (2,6-di-tert-butyl-4-methylphenol) in ethanol. The obtained granulate was dried until the temperature of the granules reached 35°C and sieved using a 1 mm sieve. To the obtained granules the extragranular part of polyethylene oxide (average molecular weight: 7,000,000) and colloidal silicon dioxide were admixed to obtain a pre-mix. Then, magnesium stearate was added to the pre-mix to obtain a mixture ready for tableting. The obtained mixture was compressed on a high speed rotary machine to yield round tablets having a diameter of 8 mm and a hardness of 100 N.

## Claims

1. Solid pharmaceutical composition comprising
(a) granules comprising (i) at least one drug selected from tamsulosin or a pharmaceutically acceptable salt or solvate thereof, and (ii) hydrogel-forming polymer, and
(b) an extragranular phase comprising (iii) hydrogel-forming polymer.

2. Solid pharmaceutical composition according to claim 1 comprising
(a) granules comprising (i) at least one drug selected from tamsulosin or a pharmaceutically acceptable salt or solvate thereof, and (ii) polyethylene oxide, and
(b) an extragranular phase comprising (iii) polyethylene oxide.

3. Composition according to claim 1 or 2, wherein the drug (a)(i) is tamsulosin hydrochloride.

4. Composition according to claim 2 or 3, wherein the polyethylene oxide (ii) and/or the polyethylene oxide (iii) have an average molecular weight of at least 100,000, preferably of at least 600,000, more preferably of at least 2,000,000 and most preferably of about 7,000,000 to about 10,000,000.

5. Composition according to any one of claims 2 to 4, wherein the weight ratio of polyethylene oxide (ii) to polyethylene oxide (iii) is about 1:200 to about 1:3, preferably about 1:100 to about 1:10, more preferably 1:50 to about 1:20.

6. Composition according to any one of claims 1 to 5, wherein granules (a) and/or the extragranular phase (b) comprise additional excipients.

7. Composition according to claim 6, wherein the additional excipients are selected from stabilizing agents, lubricants and glidants.

8. Composition according to claim 7, wherein the granules (a) comprise at least one stabilizing agent selected from iron oxides, butylated hydroxytoluene, butylated hydroxytoluene, ascorbic acid, gallic acid, caffeic acid, flavonoids, isoflavonoids such as rutin and quercetin, or mixtures thereof.

9. Composition according to claim 7 or 8, wherein the extragranular phase (b) comprises at least one lubricant selected from stearic acid, stearic acid salts such as magnesium stearate, palmitic acid salts such as magnesium palmitate, oleic acid salts such as magnesium oleate, sodium lauryl sulfate, magnesium lauryl sulfate, hydrogenated vegetable oil, hydrogenated castor oil, talc, sodium stearyl fumarate, macrogols or mixtures thereof.

10. Composition according to any one of claims 7 to 9, wherein the extragranular phase (b) comprises at least one glidant selected from tribasic calcium phosphate, powdered cellulose, colloidal silicon dioxide, magnesium oxide, magnesium silicate, magnesium trisilicate, starch, talc or mixtures thereof.

11. Composition according to any one of claims 1 to 10 comprising at least one coating.

12. Process for preparing a solid pharmaceutical composition according to any one of claims 1 to 11 comprising
(a) granulating (i) at least one drug selected from tamsulosin or a pharmaceutically acceptable salt or solvate thereof, (ii) hydrogel-forming polymer, preferably polyethylene oxide, and optionally further excipients to obtain granules,
(b) mixing the granules obtained in step (a) with (iii) hydrogel-forming polymer, preferably polyethylene oxide, and optionally further excipients to obtain a mixture,
(c) compressing the mixture obtained in step (b) to form a solid pharmaceutical composition, and
(d) optionally coating the solid pharmaceutical composition obtained in step (c) with a coating material.

13. Process according to claim 12, wherein step (a) is carried out by wet granulation, preferably by using water, an organic solvent such as ethanol or a mixture thereof as granulation liquid.

14. Process according to claim 12 or 13, wherein in step (b) the granules and hydrogel-forming polymer (iii), preferably polyethylene oxide, are firstly mixed with a glidant to obtain a pre-mix and said pre-mix is then mixed with a lubricant to obtain the mixture to be compressed in step (c).

15. Process according to any one of claims 12 to 14, wherein in step (c) the mixture is compressed using a compression pressure of about 1 to about 30 kN.

16. Solid pharmaceutical composition obtainable by a process according to any one of claims 12 to 15.
